# EUROPEAN PATENT APPLICATION

(11) **EP 4 501 943 A1**
(43) Date of publication of application: **05.02.2025**
(21) Application number: 22933740.7
(22) Date of filing: 15.04.2022
(51) Int. Cl.: C07K 7/06, A61P 3/10, A61K 38/00, A23L 33/18

(54) **PEPTIDE HAVING ANTIDIABETIC ACTIVITY, PEPTIDE COMPLEX, AND USE THEREOF**

(30) Priority: 24.03.2022 KR 20220036947
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR); KIM, Seon Soo, Anyang-si, Gyeonggi-do 14119 (KR)
(74) Representative: Heinonen & Co
(86) International application number: PCT/KR2022/005466
(87) International publication number: WO 2023/182567

(57) **Abstract**

The present invention relates to a peptide complex having an antidiabetic activity and to a use thereof. The peptide complex of the present invention promotes uptake of glucose into cells, suppresses insulin resistance signals, promotes insulin sensitivity signals, and suppresses the death of pancreatic beta cells, which are insulin-producing cells, thereby exhibiting an effect of lowering blood glucose levels. In addition, the present invention relates to a peptide having an antidiabetic activity and an antiobesity activity and to a use thereof. The peptide of the present invention has an activity of suppressing insulin resistance signals, promoting insulin sensitive signals, and promoting fat decomposition in adipocytes.

## Description

### BACKGROUND OF THE INVENTION

### Field of the Invention

The present invention relates to a peptide complex having anti-diabetic activity and uses thereof. In addition, the present invention relates to a peptide having anti-obesity and anti-diabetic activities and uses thereof.

### Description of the Related Art

Diabetes is a type of metabolic disease wherein impaired insulin secretion or absence of normal functions occurs and is characterized by hyperglycemia, that is, increased blood glucose levels, which causes various symptoms and signs and results in the discharge of glucose into the urine. In recent years, the incidence of diabetes has been an explosively growing trend due to the increased rate of obesity, particularly abdominal obesity. Diabetes can be largely divided into type 1 diabetes, which is insulin-dependent diabetes, and type 2 diabetes, which is non-insulin-dependent diabetes. Type 2 diabetes is characterized by hyperglycemia, insulin resistance, and relative impaired insulin secretion.

When food is ingested, glucose from food is absorbed from the digestive tract and stimulates insulin secretion from beta cells of the pancreas, and the secreted insulin promotes glucose absorption into muscles. Insulin is also partly involved in hepatic glucose uptake, but mainly inhibits hepatic glucose production. Insulin lowers blood glucose concentration by inhibiting glucose production in the liver and promoting glucose uptake in the peripheral tissues, including muscles. Insulin resistance refers to a condition in which a given insulin concentration produces a less than normal blood glucose response. Insulin regulates blood glucose by promoting glucose uptake in muscles or inhibiting glucose production in the liver. Insulin resistance refers to a condition in which insulin action is reduced even in the absence of insulin deficiency. Insulin receptors on the cell membrane are involved in the glucose uptake into cells of peripheral tissues, and insulin resistance occurs due to a decrease in the number of insulin receptors or an intracellular defect after receptor binding. Although insulin receptor defects are found in type 2 diabetes, it is known that mainly post-receptor intracellular defects, that is, defects in phosphorylation/dephosphorylation regulated by insulin, play a much greater role. Among these mechanisms, it is known that a phosphatidylinositol-3-kinase (PI3K) signaling defect reduces translocation of the glucose transporter GLUT-4 (glucose transporter type 4) to the cell membrane.

Nowadays, the control of blood glucose levels is accomplished by lifestyle improvement (diet therapy, exercise therapy), and medications. However, diet therapy or exercise therapy is difficult to strictly manage and practice, with limitations of the effects thereof. Hence, most patients with diabetes rely on the control of blood glucose by medications, such as insulin, insulin secretagogues, insulin sensitizers, and hypoglycemic agents, as well as lifestyle improvement.

Obesity refers to a state in which the excess energy is accumulated as body fat when food intake is not balanced with energy expenditure, and thus adipose tissue is excessively present in the body. According to the World Health Organization (WHO), more than 1 billion adults worldwide are overweight, of which at least 3 million are clinically obese, and the prevalence of obesity has increased markedly in the United States and Europe. Being overweight and obese increases blood pressure and cholesterol levels, leading to various diseases, such as heart disease, diabetes, and arthritis, and raises the incidence of various adult diseases. In addition, overweight and obesity are factors that increase the incidence of various adult diseases, such as arteriosclerosis, hypertension, hyperlipidemia, or heart disease, even in children and adolescents as well as adults.

Currently, the representative anti-obesity medications approved by the US FDA and widely prescribed include a group of drugs that act on the central nervous system to suppress appetite and orlistat (Xenical), an inhibitor of the digestive enzyme lipase secreted from the pancreas. Regarding drugs acting on the central nervous system, a number of drugs, such as sibutramine, have been revoked due to cardiovascular and psychiatric side effects. Orlistat has limitations in that it has various side effects, and the drug effect is variable depending on the amount of fat intake. Meanwhile, as an endocrine peptide targeting drug, liraglutide, a glucagon-like peptide-1 (GLP-1) receptor promoter, has been approved and used, but the risk of thyroid cancer is rising.

### [Prior art documents]

### [Patent Documents]

(Patent Document 1) WO2016-175362
(Patent Document 2) WO2018-074682

### SUMMARY OF THE INVENTION

### Technical Problems

The present inventors have conducted studies and made efforts to find an active substance with improved efficacy and secured safety for the treatment of diabetes in terms of promoting glucose uptake due to inhibition of insulin resistance and increased insulin sensitivity, and protecting pancreatic beta cells from free fatty acids. Consequently, the present inventors experimentally found that a complex of two types of peptides and a peptide having a novel amino acid sequence satisfy the above requirements and have completed the present invention.

Accordingly, one object of the present invention is to provide a peptide complex having anti-diabetic activity.

Another object of the present invention is to provide a pharmaceutical composition for preventing or treating diabetes, the composition comprising the peptide complex having the above-described activity as an active ingredient.

Another object of the present invention is to provide a functional food composition for controlling blood glucose levels, the composition comprising the peptide complex having the above-described activity as an active ingredient.

Another object of the present invention is to provide a novel peptide having anti-diabetic and anti-obesity activities.

Another object of the present invention is to provide a pharmaceutical composition and a functional food composition, for preventing, treating, or alleviating diabetes, the composition comprising the novel peptide having the above-described activities as an active ingredient.

Another object of the present invention is to provide a pharmaceutical composition and a functional food composition, for preventing, treating, or alleviating obesity, the composition comprising the novel peptide having the above-described activities as an active ingredient.

### Technical Solutions

According to an aspect of the present invention,
there is provided a peptide complex comprising: (i) a peptide comprising the amino acid sequence of SEQ ID NO: 1; and (ii) a peptide comprising the amino acid sequence of SEQ ID NO: 2.

In addition, according to another aspect of the present invention, there is provided a pharmaceutical composition for preventing or treating diabetes, the composition comprising the peptide complex as an active ingredient.

In addition, according to another aspect of the present invention, there is provided a functional food composition for preventing or alleviating diabetes, the composition comprising the peptide complex as an active ingredient.

In addition, according to another aspect of the present invention, there is provided a peptide comprising the amino acid sequence of SEQ ID NO: 2.

In addition, according to another aspect of the present invention, there is provided a pharmaceutical composition for preventing or treating diabetes, the composition comprising the peptide as an active ingredient.

In addition, according to another aspect of the present invention, there is provided a pharmaceutical composition for preventing or treating obesity, the composition comprising the peptide as an active ingredient.

In addition, according to another aspect of the present invention, there is provided a functional food composition for controlling blood glucose levels, the composition comprising the peptide as an active ingredient.

In addition, according to another aspect of the present invention, there is provided a functional food composition for preventing or alleviating obesity, the composition comprising the peptide as an active ingredient.

Hereinafter, the present invention will be described in detail.

### 1. Peptide, Peptide complex and their activities

According to an aspect of the present invention, there is provided a peptide complex comprising: (i) a peptide comprising the amino acid sequence set forth in SEQ ID NO: 1; and (ii) a peptide comprising the amino acid sequence set forth in SEQ ID NO: 2.

According to another aspect of the present invention, there is provided a peptide comprising the amino acid sequence set forth in SEQ ID NO: 2.

As used herein, the term "peptide" refers to a linear molecule formed by amino acid residues linked to each other via peptide linkages.

The peptide comprising the amino acid sequence of SEQ ID NO: 1 of the present invention or the peptide comprising the amino acid sequence of SEQ ID NO: 2 may be used without modification, but may be variants or fragments of amino acids having different sequences via deletion, insertion, and substitution of amino acid residues or combinations thereof within a range that does not affect the original activities of the peptides, such as the anti-diabetic activity.

The peptides of the present invention may be modified by phosphorylation, sulfation, acrylation, glycosylation, methylation, farnesylation, or the like, within a range that does not change the activities of the peptides.

The peptides of the present invention include a peptide comprising an amino acid sequence substantially identical to the peptide comprising the amino acid sequence of SEQ ID NO: 1 or the peptide comprising the amino acid sequence of SEQ ID NO: 2, and a variant thereof, or an active fragment thereof. The substantially identical amino acid sequence means an amino acid sequence having 75% or more, for example, 80% or more, 85% or more, 90% or more, 95% or more, 97% or more sequence identity to the amino acid sequence of SEQ ID NO: 1 or the amino acid sequence of SEQ ID NO: 2, respectively. In addition, the peptides may further include a targeting sequence, a tag, a labeled residue, or an amino acid sequence prepared for a specific purpose to increase half-life or peptide stability.

In order to select some regions of the amino acid sequence and increase the activities thereof, the peptides of the present invention may be modified at N- or C-terminals. Through such N-terminal and/or C-terminal modification, the stability of the peptides of the present invention can be remarkably improved. For example, the half-life can be increased when the peptides are administered in vivo. The term "stability" is meant to include storage stability (e.g., stability during storage at room temperature) as well as in vivo stability that protects the peptides of the present invention from the attack of a protein cleavage enzyme in vivo.

The N-terminal modification may be an attachment of a protecting group selected from the group consisting of an acetyl group, a fluorenyl methoxycarbonyl group, a formyl group, a palmitoyl group, a myristyl group, a stearyl group, and polyethylene glycol (PEG) to the N-terminal of the peptides. The C-terminal modification may be an attachment of a hydroxyl group (-OH), an amino group (-NH2), an azide group (-NHNH2), or the like, to the C-terminal of the peptides, but is not limited thereto.

The peptides of the present invention may be prepared using various methods well known in the art to which the present invention pertains. For example, the peptides of the present invention may be prepared using chemical synthesis methods known in the art, especially, solid-phase synthesis techniques (Merrifield, J. Amer. Chem. Soc. 85:2149-54 (1963); Stewart, et al., Solid Phase Peptide Synthesis, 2nd. ed., Pierce Chem. Co.: Rockford, 111(1984)) or liquid-phase synthesis techniques (U.S. Patent No. 5,516,891).

The peptide complex of the present invention includes a peptide comprising the amino acid sequence of SEQ ID NO: 1 and a peptide comprising the amino acid sequence of SEQ ID NO: 2.

The peptide complex of the present invention may refer to a mixture in which the above-described peptide comprising the amino acid sequence of SEQ ID NO: 1 and the peptide comprising the amino acid sequence of SEQ ID NO: 2 are mixed.

In the peptide complex of the present invention, the ratio of the peptide comprising the amino acid sequence of SEQ ID NO: 1 and the peptide comprising the amino acid sequence of SEQ ID NO: 2 is not limited to a particular range. For example, with regard to the above ratio, an appropriate range may be selected within the weight ratio range of 1:0.1-100.

The peptide complex of the present invention has anti-diabetic activity.

The peptide complex of the present invention has the activity of promoting glucose uptake into cells. The cells may be adipocytes, myocytes, or hepatocytes.

The peptide complex of the present invention has the activity of promoting the expressions of one or more genes selected from the group consisting of Leptin, Adiponectin, insulin receptor substrate 1 (IRS-1), glucose transporter type 4 (GLUT4), peroxisome proliferator-activated receptor-gamma coactivator-1 alpha (PGC-1α), acyl-CoA oxidase 1 (ACOX-1), peroxisome proliferator-activated receptor-alpha (PPAR-α), and carnitine palmitoyltransferase 1 alpha (CPT-1α), in cells.

The peptide complex of the present invention has the activity of inhibiting an insulin-resistant signal.

The peptide complex of the present invention has the activity of inhibiting phosphorylation of Ser302 of an insulin receptor substrate (IRS) or phosphorylation of c-Jun N-terminal kinase (JNK).

The peptide complex of the present invention has the activity of inhibiting the expressions of the TNF-α gene, the mammalian target of rapamycin (mTOR) gene, or the p70S6K gene under an insulin resistance-inducing environment.

The peptide complex of the present invention has the activity of promoting an insulin-sensitive signal.

The peptide complex of the present invention has the activity of enhancing phosphorylation of Tyr632 of an insulin receptor substrate (IRS) or of promoting activation of phosphoinositide 3-kinase (PI3K), activation of ATK, or activation of AMP-activated protein kinase (AMPK).

The peptide complex of the present invention has the activity of inhibiting the production of reactive oxygen species (ROS), the expression of the TNF-α gene, the expression of the TNF-α protein, or the expression of the IL-1β protein, which are induced by free fatty acids.

The peptide complex of the present invention has the activity of inhibiting apoptosis of pancreatic beta cells induced by free fatty acids.

Since the peptide complex of the present invention described above has the above-described activities, it can exhibit an excellent effect in treating diabetes.

The peptide of the present invention comprising the amino acid sequence of SEQ ID NO: 2 has anti-diabetic activity.

The peptide of the present invention comprising the amino acid sequence of SEQ ID NO: 2 has the activity of inhibiting an insulin-resistant signal or promoting an insulin-sensitive signal.

Specifically, the peptide of the present invention comprising the amino acid sequence of SEQ ID NO: 2 can promote an insulin-sensitive signal through the activity of promoting phosphorylation of Tyr632 of an insulin receptor substrate (IRS), the activity of promoting activation of phospho-AKT, or the activity of promoting phosphorylation of AMPK. The peptide of the present invention comprising the amino acid sequence of SEQ ID NO: 2 can inhibit an insulin-resistant signal through the activity of inhibiting phosphorylation of Ser302 of an IRS under an insulin resistance-inducing environment.

The peptide of the present invention comprising the amino acid sequence of SEQ ID NO: 2 has anti-obesity activity.

The peptide of the present invention comprising the amino acid sequence of SEQ ID NO: 2 has the activity of promoting lipolysis in adipocytes.

Specifically, the peptide of the present invention comprising the amino acid sequence of SEQ ID NO: 2 can increase the expressions of adipose triglyceride lipase (ATGL), phosphorylated hormone-sensitive lipase (pHSL), or perilipin (PLIN, lipid droplet-associated protein), which are lipolytic enzyme proteins, in adipocytes.

Since the peptide of the present invention comprising the amino acid sequence of SEQ ID NO: 2 described above has the above-described activities, it can exhibit excellent effects in treating, preventing, or alleviating diabetes and obesity.

### 2. Compositions for preventing, treating, or alleviating diabetes

### Pharmaceutical compositions

According to another aspect of the present invention, there is provided a pharmaceutical composition for preventing or treating diabetes, the composition comprising, as an active ingredient, a peptide complex comprising: (i) a peptide comprising the amino acid sequence of SEQ ID NO: 1; and (ii) a peptide comprising the amino acid sequence of SEQ ID NO: 2.

As described above, the peptide complex of the present invention has glucose uptake-promoting activity, insulin resistance-inhibiting activity, insulin sensitivity-promoting activity, and pancreatic beta cell-protecting activity, and thus has excellent effects on treating or preventing diabetes.

In the present invention, diabetes may be type 1 diabetes or type 2 diabetes, specifically type 2 diabetes.

In the pharmaceutical composition for preventing or treating diabetes, the peptide complex may promote glucose uptake.

In the pharmaceutical composition for preventing or treating diabetes, the peptide complex may inhibit an insulin-resistant signal or promote an insulin-sensitive signal.

In the pharmaceutical composition for preventing or treating diabetes, the peptide complex may inhibit phosphorylation of Ser302 of an insulin receptor substrate (IRS) or phosphorylation of c-Jun N-terminal kinase (JNK).

In the pharmaceutical composition for preventing or treating diabetes, the peptide complex may inhibit the expressions of the TNF-α gene, the mammalian target of rapamycin (mTOR) gene, or the p70S6K gene under an insulin resistance-inducing environment.

In the pharmaceutical composition for preventing or treating diabetes, the peptide complex may enhance phosphorylation of Tyr632 of an insulin receptor substrate (IRS) or promote activation of phosphoinositide 3-kinase (PI3K), activation of AKT, or activation of AMP-activated protein kinase (AMPK).

In the pharmaceutical composition for preventing or treating diabetes, the peptide complex may promote the expressions of one or more genes selected from the group consisting of Leptin, Adiponectin, insulin receptor substrate 1 (IRS-1), glucose transporter type 4 (GLUT4), PGC-1α, ACOX-1, PPAR-α, and CPT-1α.

In the pharmaceutical composition for preventing or treating diabetes, the peptide complex may inhibit the production of reactive oxygen species (ROS), the expression of the TNF-α gene, the expression of the TNF-α protein, or the expression of the IL-1β protein, which are induced by free fatty acids, or may inhibit apoptosis of pancreatic beta cells induced by free fatty acids.

According to another aspect of the present invention, there is provided a pharmaceutical composition for preventing or treating diabetes, the composition comprising a peptide comprising the amino acid sequence of SEQ ID NO: 2 as an active ingredient.

Since the peptide of the present invention comprising the amino acid sequence of SEQ ID NO: 2 has the activity of inhibiting an insulin-resistant signal and the activity of promoting insulin sensitivity as described above, it has excellent effects in treating or preventing diabetes.

In the present invention, diabetes may be type 1 diabetes or type 2 diabetes, specifically type 2 diabetes.

In the pharmaceutical composition for preventing or treating diabetes, the peptide may inhibit an insulin-resistant signal or promote an insulin-sensitive signal.

In the pharmaceutical composition for preventing or treating diabetes, the peptide may promote phosphorylation of Tyr632 of an insulin receptor substrate (IRS), promote activation of phospho-AKT, or promote phosphorylation of AMPK.

In the pharmaceutical composition for preventing or treating diabetes, the peptide may inhibit phosphorylation of Ser302 of an IRS under an insulin resistance-inducing environment.

According to another aspect of the present invention, there is provided a pharmaceutical composition for preventing or treating obesity, the composition comprising a peptide comprising the amino acid sequence of SEQ ID NO: 2 as an active ingredient.

In the pharmaceutical composition for preventing or treating obesity, the peptide may promote lipolysis in adipocytes.

In the pharmaceutical composition for preventing or treating obesity, the peptide can increase the expressions of adipose triglyceride lipase (ATGL), phosphorylated hormone-sensitive lipase (pHSL), or perilipin (PLIN, lipid droplet-associated protein), which are lipolytic enzyme proteins, in adipocytes.

The pharmaceutical composition of the present invention may include a therapeutically effective amount of the peptide complex or of the peptide and a pharmaceutically acceptable carrier.

The term "therapeutically effective amount" used herein refers to an amount sufficient to achieve the activity or efficacy of the peptide complex or the peptide, the active ingredient of the pharmaceutical composition of the present invention, for example, an amount sufficient to achieve the efficacy of treating or preventing diabetes or obesity.

The pharmaceutically acceptable carrier is commonly used in preparation and includes, but is not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, and the like.

In addition to the above ingredients, the pharmaceutical composition of the present invention may further include a lubricant, a humectant, a sweetener, a flavoring agent, an emulsifier, a suspending agent, a preservative, and the like. However, the present invention is not limited thereto.

Suitable pharmaceutically acceptable carriers and agents are described in detail in Remington: The Science and Practice of Pharmacy, (19th ed., 1995, Williams & Wilkins).

The pharmaceutical composition of the present invention may be administered by any suitable route for treating diabetes or obesity, for example, may be administered orally or parenterally. Parenteral administration may be intravenous injection, subcutaneous injection, intramuscular injection, intraperitoneal injection, topical administration, transdermal administration, and the like.

The dosage of the pharmaceutical composition may be, but not limited to, 0.0001 µg to 100 mg, 0.001 µg to 100 mg, 0.01 µg to 100 mg, 0.1 µg to 100 mg, or 1.0 µg to 1000 mg per day. The pharmaceutical composition may be prescribed in various ways depending on factors, such as preparation methods, administration mode, the patient's age, weight, gender, pathological condition, diet, the time of administration, administration route, excretion rate, and response sensitivity.

The pharmaceutical composition of the present invention may be prepared in unit dosage form or by incorporation into a multi-dose container, using a pharmaceutically acceptable carrier and/or excipient according to a method that can be easily carried out by a person skilled in the art to which the present invention pertains. Herein, the formulation may be in the form of a solution, suspension, or emulsion in oil or aqueous medium, or may be in the form of an extract, powders, granules, a tablet, or a capsule, and may further comprise a dispersing agent or a stabilizer.

### Food compositions

According to another aspect of the present invention, there is provided a functional food composition for controlling blood glucose levels, the composition comprising, as an active ingredient, a peptide complex comprising: (i) a peptide comprising the amino acid sequence of SEQ ID NO: 1; and (ii) a peptide comprising the amino acid sequence of SEQ ID NO: 2.

In the functional food composition of the present invention, the control of the blood glucose levels may be the control of the blood glucose levels in patients with diabetes or high-risk patients with pre-diabetes.

In the functional food composition of the present invention, diabetes may be type 1 diabetes or type 2 diabetes, specifically type 2 diabetes.

In the functional food composition of the present invention, the control of the blood glucose levels may be a lowering of the blood glucose levels.

In the functional food composition of the present invention, the peptide complex may be included in an appropriate amount selected within the range of 0.0001 wt% to 10 wt% based on the total weight of the composition.

According to another aspect of the present invention, there is provided a functional food composition for controlling blood glucose levels, the composition comprising a peptide comprising the amino acid sequence of SEQ ID NO: 2 as an active ingredient.

In the functional food composition of the present invention, the control of the blood glucose levels may be the control of the blood glucose levels in patients with diabetes or high-risk patients with pre-diabetes.

In the functional food composition of the present invention, diabetes may be type 1 diabetes or type 2 diabetes, specifically type 2 diabetes.

In the functional food composition of the present invention, the control of the blood glucose levels may be a lowering of the blood glucose levels.

In the functional food composition for controlling blood glucose levels, the peptide may inhibit an insulin-resistant signal or promote an insulin-sensitive signal.

In the functional food composition for controlling blood glucose levels, the peptide may promote phosphorylation of Tyr632 of an insulin receptor substrate (IRS), promote activation of phospho-AKT, or promote phosphorylation of AMPK.

In the functional food composition for controlling blood glucose levels, the peptide may inhibit phosphorylation of Ser302 of an IRS under an insulin resistance-inducing environment.

According to another aspect of the present invention, there is provided a functional food composition for preventing or alleviating obesity, the composition comprising a peptide comprising the amino acid sequence of SEQ ID NO: 2 as an active ingredient.

In the functional food composition for preventing or alleviating obesity, the peptide may promote lipolysis in adipocytes.

In the functional food composition for preventing or alleviating obesity, the peptide can increase the expressions of lipolytic enzyme proteins, adipose triglyceride lipase (ATGL), phosphorylated hormone-sensitive lipase (pHSL), or perilipin (PLIN, lipid droplet-associated protein) in adipocytes.

In the functional food composition of the present invention, the peptide may be included in an appropriate amount selected within the range of 0.0001 wt% to 10 wt% based on the total weight of the composition.

In one embodiment, the functional food composition of the present invention may include a bromatologically effective amount of the peptide complex and a bromatologically acceptable carrier.

The food composition of the present invention may include not only the peptide complex or the peptide as the active ingredient, but also ingredients commonly added during food production, for example, proteins, carbohydrates, fats, nutrients, seasonings, and flavoring agents. Examples of such carbohydrates are monosaccharides such as glucose, fructose and the like; disaccharides such as maltose, sucrose, oligosaccharides, and the like; and conventional sugars such as polysaccharides such as dextrin, cyclodextrin, and sugar alcohols such as xylitol, sorbitol, and erythritol. Examples of flavoring agents may include natural flavoring agents, thaumatin, stevia extracts (for example, rebaudioside A, glycyrrhizin, etc.), and synthetic flavoring agents (saccharin, aspartame, etc.). The ratio of the carbohydrates may be, but not limited to, about 1 to 20 g, preferably about 5 to 12 g per 100 g of the food composition of the present invention.

The functional food composition of the present invention may contain various nutrients, vitamins, minerals (electrolytes), flavoring agents, such as synthetic and natural flavoring agents, colorants and thickeners (cheese, chocolate, etc.), pectic acid and salts thereof, alginic acid and salts thereof, organic acids, protective colloidal thickeners, pH adjusters, stabilizers, preservatives, glycerin, alcohols, carbonating agents used in carbonated beverages, and the like, in addition to the above-described ingredients. In addition, it may contain pulp for preparing a natural fruit juice, a fruit juice drink, or a vegetable drink.

For example, when the food composition of the present invention is prepared as a drink, it may further comprise citric acid, liquid fructose, sugar, glucose, acetic acid, malic acid, fruit juice, a eucommia extract, a jujube extract, a licorice extract, etc., in addition to the effective ingredient, the peptide complex.

According to another aspect of the present invention, the present invention provides a method for treating diabetes, the method comprising administering to a diabetic patient a therapeutically effective amount of the above-described peptide complex comprising: (i) a peptide comprising the amino acid sequence of SEQ ID NO: 1; and (ii) a peptide comprising the amino acid sequence of SEQ ID NO: 2.

According to another aspect of the present invention, the present invention provides a method for controlling blood glucose levels, the method comprising administering to a subject in need thereof a therapeutically effective amount of the above-described peptide complex comprising: (i) a peptide comprising the amino acid sequence of SEQ ID NO: 1; and (ii) a peptide comprising the amino acid sequence of SEQ ID NO: 2.

According to another aspect of the present invention, there is provided a method for treating, preventing, or alleviating diabetes, the method comprising administering to a diabetic patient a therapeutically effective amount of a peptide comprising the amino acid sequence of SEQ ID NO: 2 described above.

According to another aspect of the present invention, there is provided a method for controlling blood glucose, comprising administering a therapeutically effective amount of a peptide comprising the amino acid sequence of SEQ ID NO: 2 described above to a subject in need thereof.

According to another aspect of the present invention, there is provided a method for treating, alleviating, or preventing obesity, the method comprising administering to a subject in need of treatment of obesity a therapeutically effective amount of a peptide comprising the amino acid sequence of SEQ ID NO: 2 described above.

### Advantageous Effects

The peptide complex or the peptide of the present invention promotes glucose uptake into cells, inhibits an insulin-resistant signal, promotes an insulin-sensitive signal, and inhibits apoptosis of pancreatic beta cells, which are insulin-producing cells, and thus can exhibit the effect of lowering the blood glucose levels. The peptide complex or the peptide of the present invention can be used for treating, preventing, and alleviating diabetes, and can be usefully used for lowering blood glucose in a patient with diabetes or a high-risk patient with pre-diabetes.

However, the effects of the present invention are not limited to the above-mentioned effects and other effects not mentioned will be clearly understood from the following description by a person skilled in the art.

### BRIEF DESCRIPTION OF THE DRAWINGS

Figures 1a and 1b are experimental results showing that the peptide complex of the present invention promotes glucose uptake into adipocytes.
Figures 2a and 2b are experimental results showing that the peptide complex of the present invention promotes glucose uptake into myoblasts.
Figures 3a and 3b are experimental results showing that the peptide complex of the present invention has the effects of promoting the activities of phospho-AMPK and phospho-ACC, which are insulin sensitivity-promoting factors.
Figures 4a and 4b are experimental results showing that the peptide complex of the present invention has the effects of inhibiting phosphorylations of IRS Serine302 and JNK, which are insulin resistance-signaling factors.
Figures 5a and 5b are experimental results showing that the peptide complex of the present invention again reduces the increases in the gene expressions of the insulin resistance-inducing cytokine TNF-α and the insulin resistance-promoting signaling factors mTOR and p70S6K, induced by rhTNF-α treatment.
Figures 6a and 6b are experimental results showing that when adipocytes in which insulin resistance had been induced by rhTNF-α treatment were treated with the peptide complex of the present invention, phosphorylation of IRS Tyrosine632, an insulin sensitivity-promoting factor, was enhanced, and activations of PI3K, ATK, and AMPK were promoted.
Figures 7a and 7b are experimental results showing that the expressions of glucose uptake-related genes of leptin, adiponectin, IRS-1, and GLUT4, which had been decreased by rhTNF-α treatment, in adipocytes, were increased by treatment with the peptide complex of the present invention.
Figures 8a and 8b are experimental results showing that palmitate-induced reactive oxygen species (ROS) were reduced by the peptide complex of the present invention.
Figures 9a and 9b are experimental results showing that the gene expression of TNF-α, an inflammatory protein induced by palmitate, was significantly reduced by treatment with the peptide complex of the present invention.
Figures 10a and 10b are experimental results showing that the increased expression levels of the TNF-α and the IL-1β proteins by palmitate treatment in INS-1 cells (rat pancreatic beta cells) were again significantly reduced by treatment with the peptide complex of the present invention.
Figure 11 is an experimental result showing that the increase in INS-1 cell death induced by palmitate treatment was again reduced by treatment with the peptide complex of the present invention, thereby increasing the cell viability.
Figures 12a and 12b are experimental results showing that the expression of genes of PGC-1α, ACOX-1, PPAR-α, or CPT-1α, which are genes related to FFA beta-oxidation, was promoted by the peptide complex of the present invention in HepG2 cells.
Figures 13a and 13b show the activity of the peptide of the present invention of increasing the expressions of lipolytic enzyme proteins, adipose triglyceride lipase (ATGL), phosphorylated hormone-sensitive lipase (pHSL), and perilipin (PLIN, lipid droplet-associated protein) in adipocytes.
Figure 14 shows a tendency in which the release amount of glycerol, which is a lipolysis product, increases in a treated peptide concentration-dependent manner when adipocytes are treated with the peptide of the present invention.
Figures 15a and 15b show that when adipocytes are treated with the peptide of the present invention, phosphorylation of Tyr632 of an insulin receptor substrate (IRS), which is a factor for promoting insulin sensitivity, and activation of phosphorylation-AKT are promoted, and phosphorylation of AMPK, a signaling protein, increases. Meanwhile, figures 15a and 15b show that phosphorylation of Ser302 of an insulin receptor substrate (IRS) decreases under an insulin resistance-inducing environment.

### DETAILED DESCRIPTION OF THE PREFERRED EMBODIMENT

Hereinafter, the present invention will be described in detail through Examples. However, the following Examples specifically illustrate the present invention, and the contents of the present invention are not limited by the following Examples.

### Examples

### Preparation Example 1: Preparation of peptides and peptide complex

The peptide having the amino acid sequence of SEQ ID NO: 1 and the peptide having the amino acid sequence of SEQ ID NO: 2 described in Table 1 below were synthesized by an automatic peptide synthesizer (Milligen 9050, Millipore, USA). Those synthesized peptides were purely separated by a C18 reversed-phase high-performance liquid chromatography (HPLC) (Waters Associates, USA). The column ACQUITY UPLC BEH300 C18 (2.1 mm X 100 mm, 1.7 µm, Waters Co, USA) was used.

**[Table 1]**

| SEQ ID NO: | The amino acid sequence of the peptide |
|---|---|
| 1 | LKTRN |
| 2 | KGSATGWMA |

Equal amounts of the prepared peptide of SEQ ID NO: 1 and the peptide of SEQ ID NO: 2 were mixed to prepare a peptide complex, and its efficacy was evaluated. In addition, the effects of the prepared peptide of SEQ ID NO: 2 were also evaluated.

### Experimental Example 1: Promotion of glucose uptake into adipocytes

Whether the peptide complex prepared in Preparation Example 1 promotes glucose uptake into cells was tested using adipocytes.

When 3T3-L1 preadipocytes reached 80% confluence, they were seeded in a 96-well plate at 5 x 10³ cells/well and used for the experiment. Two days after reaching confluence, the culture medium was replaced with a differentiation induction medium, and differentiation into adipocytes was induced. Two days after induction, preadipocytes were cultured for two days in a medium containing 10% FBS and 10 µg/mL insulin, and then the medium was replaced with a medium containing 10% FBS every two days until preadipocytes differentiated into adipocytes. When differentiation was completed, the medium was replaced with a glucose-free medium, and cells were cultured for 16 hours, followed by cultivation for 40 minutes in 100 µL of KRPH buffer containing 2% BSA to induce starvation. The cells were treated with rhTNF-α (2 nM), insulin (10 µg/ml), and the peptide complex (2 µg/ml, 20 ug/ml), cultured for one hour, treated with 80 µM 2-NBDG, and cultured for one hour, and thus a sample was prepared. Glucose uptake was measured using a "glucose uptake assay kit" (Abcam, Cambridge, UK), and the glucose absorbed into the cells was photographed with a fluorescence microscope (ECLIPSE 80i, Nikon, Japan).

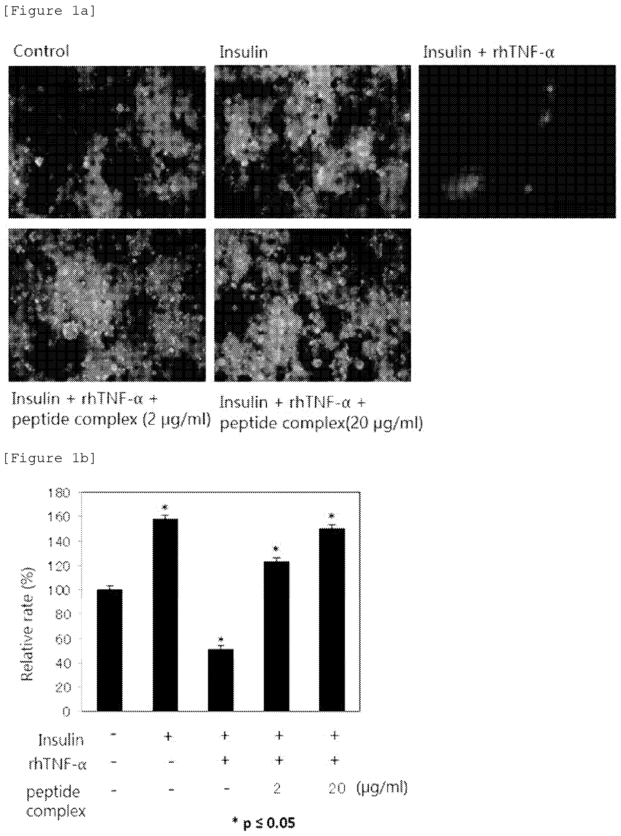

As shown in figures 1a and 1b, the experiment showed that the glucose uptake into cells that had been inhibited by TNF-α was increased by the peptide complex treatment. In addition, it was confirmed that this action occurs through the AMP-activated protein kinase (AMPK) and acetyl-CoA carboxylase (ACC) signaling pathways in Experimental Example 3 described below. These experimental results showed that the peptide complex significantly increased again the glucose uptake into cells which had been reduced by inflammation.

### Experimental Example 2: Promotion of glucose uptake into myocytes

Whether the peptide complex prepared in Preparation Example 1 promotes glucose uptake into cells was tested using myoblasts.

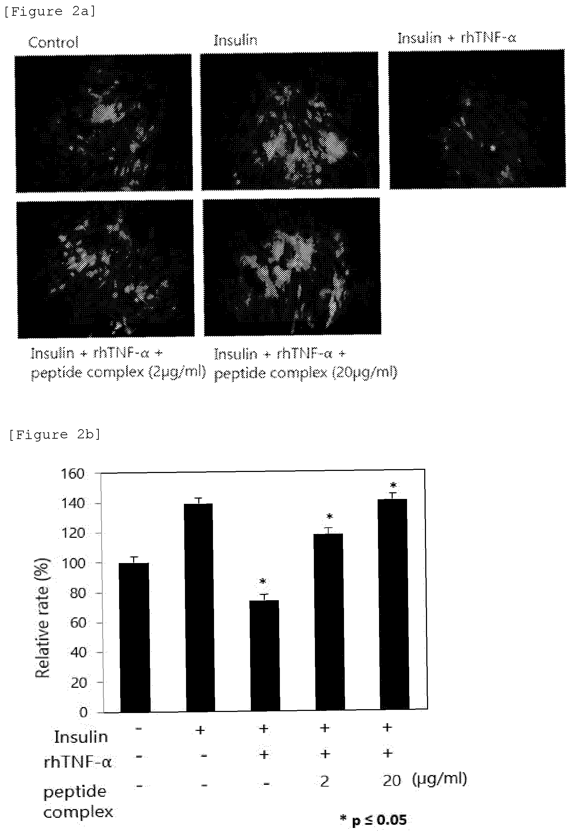

C2C12 myoblasts were seeded in a 96-well plate at 5 x 10³ cells/well and cultured in a DMEM medium containing 10% FBS for two days. The medium was replaced with a DMEM medium containing 2% horse serum, and C2C12 myoblasts were cultured for 6 days to induce differentiation into myotubes. The cultured myotubes were treated with rhTNF-α (2 nM), insulin (10 µg/ml), and the peptide complex (2 µg/ml, 20 ug/ml), cultured for one hour, treated with 80 µM 2-NBDG, and cultured for one hour, and thus a sample was prepared. Glucose uptake was measured using a "glucose uptake assay kit" (Abcam, Cambridge, UK), and the glucose absorbed into the cells was photographed with a fluorescence microscope (ECLIPSE 80i, Nikon, Japan). As shown in figures 2a and 2b, the experiment showed that the glucose uptake into myoblasts inhibited by the rhTNF-α treatment was increased by the peptide complex of the present invention.

### Experimental Example 3: Signaling pathway of the peptide complex in the glucose uptake-promoting action

Whether the peptide complex of Preparation Example 1 has the effect of promoting the activity of the insulin sensitivity-promoting factor was confirmed through protein expression analysis (western blot analysis).

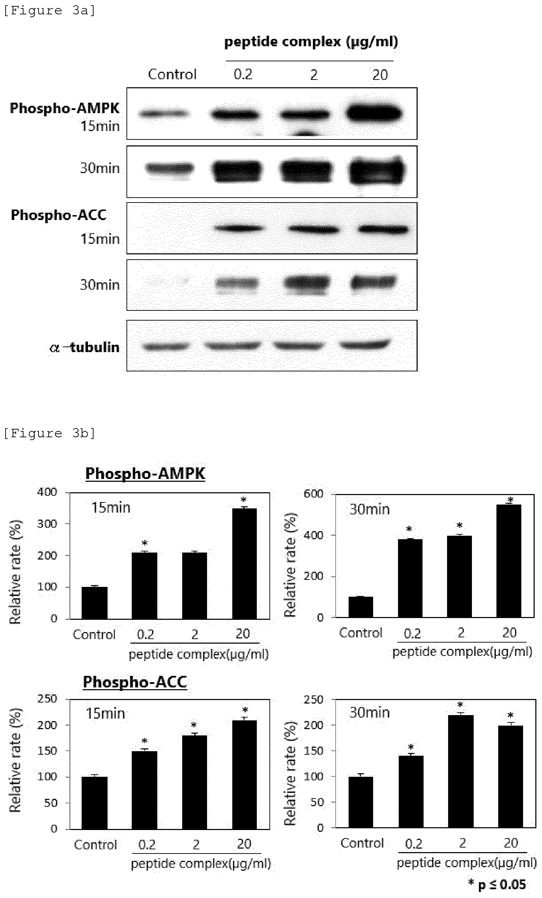

When 3T3-L1 preadipocytes reached 80% confluence, they were seeded in a 96-well plate at 5 x 10³ cells/well and used for the experiment. Two days after reaching confluence, the culture medium was replaced with a differentiation induction medium, and differentiation into adipocytes was induced. Two days after induction, preadipocytes were cultured for two days in a medium containing 10% FBS and 10 µg/mL insulin, and then the medium was replaced with a medium containing 10% FBS every two days until preadipocytes differentiated into adipocytes. When differentiation was completed, the medium was replaced with a serum-free medium, and cells were cultured for 4 hours to induce starvation. To the cells, the peptide complex (0.2, 2, and 20 µg/ml) of Preparation Example 1 was added, the cells were cultured for 30 minutes, and a lysis buffer was added to lyse the cells. Then, the lysates was centrifuged at 4°C and 12,000 rpm for 30 minutes. Thus obtained proteins were quantified using the BCA kit. Proteins were subjected to sodium dodecyl sulfate-polyacrylamide gel electrophoresis (SDD-PAGE) and electrotransferred to a membrane. The protein-attached membrane was blocked with 5% skim milk treatment, and then the primary antibody was reacted overnight at 4°C. The membrane was washed with PBS-T, the secondary antibody was reacted at room temperature for one hour, the membrane was washed again with PBS-T, and then visualization was carried out by using Gel Doc (Bio-Rad, Hercules, CA, USA) with a Western detection reagent (Elpis Biotech, Daejeon, Korea). The antibodies used in the experiment are as follows: anti-phospho-AMPK antibody (Cell signaling technology (CST), USA), anti-phospho-ACC antibody (Cell signaling technology (CST), USA), and anti-α-tubulin antibody (Santa Cruz Biotechnology). As shown in figures 3a and 3b, the experiment showed that the peptide complex of the present invention has the effects of promoting the activities of phospho-AMPK and phospho-ACC, which are insulin sensitivity-promoting factors.

### Experimental Example 4: Inhibition of an insulin-resistant signal by the peptide complex

Whether the peptide complex prepared in Preparation Example 1 has the effect of inhibiting an insulin-resistant signal was confirmed through protein expression analysis (western blot analysis).

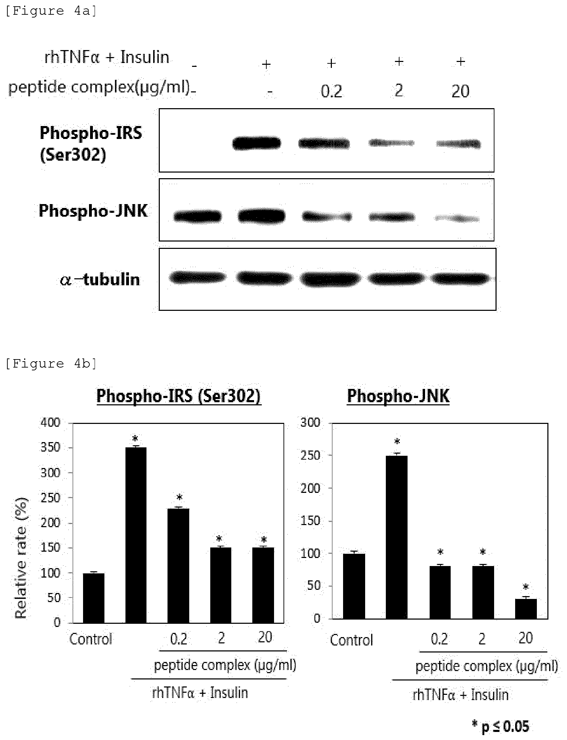

When 3T3-L1 preadipocytes reached 80% confluence, they were seeded in a 6-well plate at 5 x 10⁵ cells/well and used for the experiment. Two days after reaching confluence, the culture medium was replaced with a differentiation induction medium, and differentiation into adipocytes was induced. Two days after induction, preadipocytes were cultured for two days in a medium containing 10% FBS and 10 µg/mL insulin, and then the medium was replaced with a medium containing 10% FBS every two days until preadipocytes differentiated into adipocytes. When differentiation was completed, the medium was replaced with a serum-free medium, and cells were cultured for 4 hours to induce starvation. The cells were treated with rhTNF-α (2nM), insulin (10 µg/ml), and the peptide complex (0.2, 2, and 20 µg/ml) and cultured for 30 minutes, and a lysis buffer was added to the cells of each treatment group to lyse the cells. Then, the lysate was centrifuged at 4°C and 12,000 rpm for 30 minutes. Thus obtained proteins were quantified using the BCA kit. Proteins were subjected to sodium dodecyl sulfate-polyacrylamide gel electrophoresis (SDS-PAGE) and electrotransferred to a membrane. The protein-attached membrane was blocked with 5% skim milk treatment, and then the primary antibody was reacted overnight at 4°C. The membrane was washed with PBS-T, the secondary antibody was reacted at room temperature for one hour, the membrane was washed again with PBS-T, and then visualization was carried out by using Gel Doc (Bio-Rad, Hercules, CA, USA) with a western detection reagent (Elpis Biotech, Daejeon, Korea). The antibodies used in the experiment are as follows: anti-phospho-IRS (Ser302) antibody (Cell signaling technology (CST), USA), anti-phospho-JNK antibody (Santa Cruz Biotechnology, USA), and anti-α-tubulin antibody (Santa Cruz Biotechnology , USA). As shown in figures 4a and 4b, the experiment showed that the peptide complex of the present invention inhibited phosphorylations of Serine302 of an insulin receptor substrate (IRS) and c-Jun N-terminal kinase (JNK), which are insulin resistance-signaling factors and whose phosphorylations had been induced by TNF-α treatment.

### Experimental Example 5: Inhibition of the expressions of insulin resistance-inducing genes by the peptide complex

Whether the peptide complex prepared in Preparation Example 1 inhibits the expressions of insulin resistance-inducing genes was tested. Adipocytes were treated with rhTNF-α to induce insulin resistance, the adipocytes were treated with the peptide complex, and then the expression levels of the mTOR and p70S6K genes were measured.

When 3T3-L1 preadipocytes reached 80% confluence, they were seeded in a 6-well plate at 5 x 10⁵ cells/well and used for the experiment. Two days after reaching confluence, the culture medium was replaced with a differentiation induction medium, and differentiation into adipocytes was induced. Two days after induction, preadipocytes were cultured for two days in a medium containing 10% FBS and 10 µg/mL insulin, and then the medium was replaced with a medium containing 10% FBS every two days until preadipocytes differentiated into adipocytes. When differentiation was completed, the medium was replaced with a serum-free medium, and cells were cultured for 4 hours to induce starvation. The cells were treated with rhTNF-α (2 nM), insulin (1 µg/ml), and the peptide complex (0.2, 2, and 20 µg/ml). Twenty-four hours later, RNA was extracted from an untreated control group and the treated group, and RT-PCR was carried out. RNA was extracted from 3T3-L1 cells using the easy-BLUE^{™} Total RNA Extraction Kit (Qiagen, Germany). The extracted RNA was converted into cDNA using RT-PCR premix (iNtRON Biotechnology, Seongnam, Korea). A reaction mixture was prepared using PCR premix (iNtRON Biotechnology, Seongnam, Korea) and a primer for TNF-α, mammalian target of rapamycin (mTOR), p70S6K, or GAPDH genes, and PCR was carried out using PCR machine (Eppendorf, Germany). Subsequently, mRNA expression patterns were determined by agarose gel electrophoresis. The nucleotide sequences of the primers used in the experiment are shown in Table 2 below.

**[Table 2]**

| Gene | Primer | Sequence (5' -> 3') | SEQ ID NO: |
|---|---|---|---|
| TNF-α | F | CGT CAG CCG ATT TGC TAT CT | 3 |
| | R | CGG ACT CCG CAA AGT CTA AG | 4 |
| mTOR | F | TTG AGG TCG CTA TGA CCA GAG AGA A | 5 |
| | R | TTA CCA GAA GGG ACA CCA GCC AAT G | 6 |
| p70S6K | F | GGA GCC TGG GAG CCC TGA TGT | 7 |
| | R | GAA GCC CTC TTT GAT GCT GTC C | 8 |
| GAPDH | F | GTG ATG GCA TGG ACT GTG GT | 9 |
| | R | GGA GCC AAA AGG GTC ATC AT | 10 |

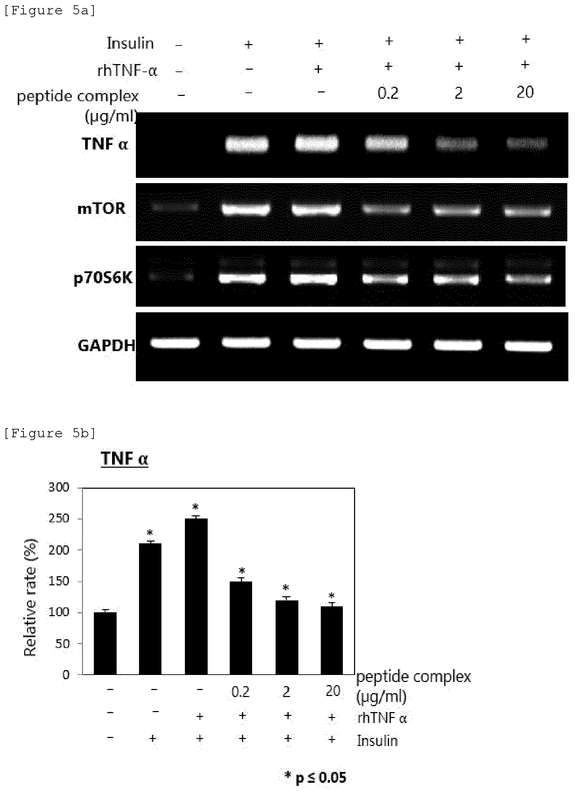

As shown in figures 5a and 5b, the experiment showed that the rhTNF-α treatment increased the expressions of the TNF-α gene, an insulin resistance-inducing inflammatory cytokine, and mTOR and p70S6K genes, insulin resistance-promoting signaling factors, but the expressions of the genes were reduced again with the peptide complex treatment.

### Experimental Example 6: Promotion of an insulin-sensitive signal by the peptide complex

Whether the peptide complex prepared in Preparation Example 1 promotes an insulin-sensitive signal was tested.

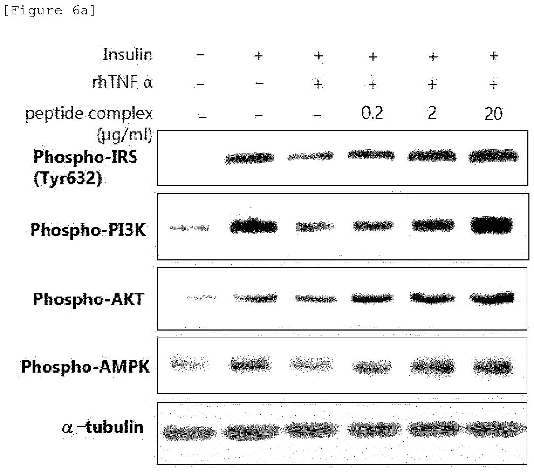

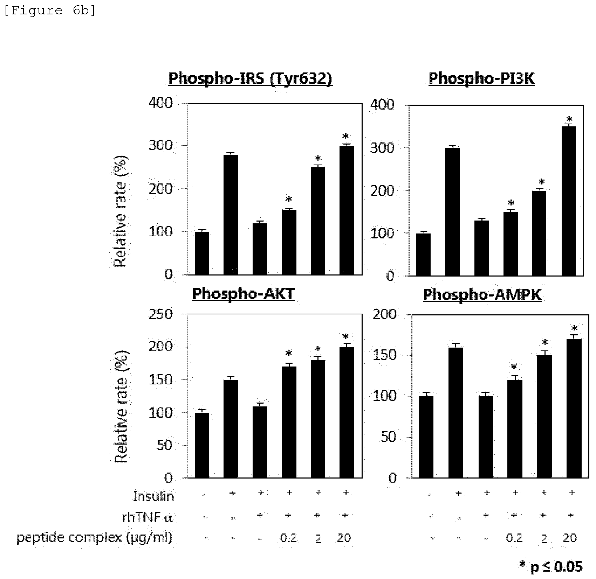

When 3T3-L1 preadipocytes reached 80% confluence, they were seeded in a 6-well plate at 5 x 10⁵ cells/well and used for the experiment. Two days after reaching confluence, the culture medium was replaced with a differentiation induction medium, and differentiation into adipocytes was induced. Two days after induction, preadipocytes were cultured for two days in a medium containing 10% FBS and 10 µg/mL insulin, and then the medium was replaced with a medium containing 10% FBS every two days until preadipocytes differentiated into adipocytes. When differentiation was completed, the medium was replaced with a serum-free medium, and cells were cultured for 4 hours to induce starvation. The cells were treated with rhTNF-α (2nM), insulin (1 µg/ml), and the peptide complex (0.2, 2, and 20 µg/ml) and cultured for 30 minutes, and a lysis buffer was added to the cells of each treatment group to lyse the cells. Then, the lysate was centrifuged at 4°C and 12,000 rpm for 30 minutes. Thus obtained proteins were quantified using the BCA kit. Proteins were subjected to sodium dodecyl sulfate-polyacrylamide gel electrophoresis (SDS-PAGE) and electrotransferred to a membrane. The protein-attached membrane was blocked with 5% skim milk treatment, and then the primary antibody was reacted overnight at 4°C. The membrane was washed with PBS-T, the secondary antibody was reacted at room temperature for one hour, the membrane was washed again with PBS-T, and then visualization was carried out by using Gel Doc (Bio-Rad, Hercules, CA, USA) with a western detection reagent (Elpis Biotech, Daejeon, Korea). The antibodies used in the experiment are as follows: anti-phospho-IRS (Tyr632) antibody (Cell signaling technology (CST), USA), anti-phospho-PI3K antibody (Cell signaling technology(CST), USA), anti-phospho-AKT antibody (Cell signaling technology(CST), USA), anti-phospho-AMPK antibody (Cell signaling technology (CST), USA), and anti-α-tubulin antibody (Santa Cruz Biotechnology , USA). As shown in figures 6a and 6b, the experiment showed that in adipocytes having insulin resistance induced by rhTNF-α treatment, the peptide complex treatment increased phosphorylation of an insulin receptor substrate (IRS) Tyrosine632, an insulin sensitivity-promoting factor, and accordingly, promoted the activations of phosphoinositide 3-kinase (PI3K), AKT, and AMP-activated protein kinase (AMPK).

### Experimental Example 7: Promotion of the expressions of glucose uptake-related genes

Whether the peptide complex prepared in Preparation Example 1 promotes the expressions of glucose uptake-related genes was confirmed through the expression analysis of leptin, adiponectin, insulin receptor substrate 1 (IRS-1), and glucose transporter type 4 (GLUT4) genes.

When 3T3-L1 preadipocytes reached 80% confluence, they were seeded in a 6-well plate at 5 x 10⁵ cells/well and used for the experiment. Two days after reaching confluence, the culture medium was replaced with a differentiation induction medium, and differentiation into adipocytes was induced. Two days after induction, preadipocytes were cultured for two days in a medium containing 10% FBS and 10 µg/mL insulin, and then the medium was replaced with a medium containing 10% FBS every two days until preadipocytes differentiated into adipocytes. When differentiation was completed, the medium was replaced with a serum-free medium, and cells were cultured for 4 hours to induce starvation. The cells were treated with rhTNF-α (2 nM), insulin (1 µg/ml), and the peptide complex (0.2, 2, and 20 µg/ml). Twenty-four hours later, RNA was extracted from an untreated control group and the treated group, and RT-PCR was carried out. RNA was extracted from 3T3-L1 cells using the easy-BLUE^{™} Total RNA Extraction Kit (Qiagen, Germany). The extracted RNA was converted into cDNA using RT-PCR premix (iNtRON Biotechnology, Seongnam, Korea). A reaction mixture was prepared using PCR premix (iNtRON Biotechnology, Seongnam, Korea) and a primer for each gene, and PCR was carried out using PCR machine (Eppendorf, Germany). Subsequently, mRNA expression patterns were determined by agarose gel electrophoresis. The nucleotide sequences of the primers used in the experiment are shown in Table 3 below.

**[Table 3]**

| Gene | Primer | Sequence (5' -> 3') | SEQ ID NO: |
|---|---|---|---|
| Leptin | F | GGA TCA GGT TTT GTG GTG CT | 11 |
| | R | TTG TGG CCC ATA AAG TCC TC | 12 |
| Adiponectin | F | TCC TGC TTT GGT CCC TCC AC | 13 |
| | R | TCT CCA GCC CCA CAC TGA AC | 14 |
| IRS-1 | F | GCC AAT CTT CAT CCA GTT GC | 15 |
| | R | CAT CGT GAA GAA GGC ATA GG | 16 |
| GAPDH | F | GTG ATG GCA TGG ACT GTG GT | 9 |
| | R | GGA GCC AAA AGG GTC ATC AT | 10 |
| GLUT4 | F | ACT AAG AGC ACC GAG ACC AA | 27 |
| | R | CTG CCC GAA AGA GTC TAA AG | 28 |

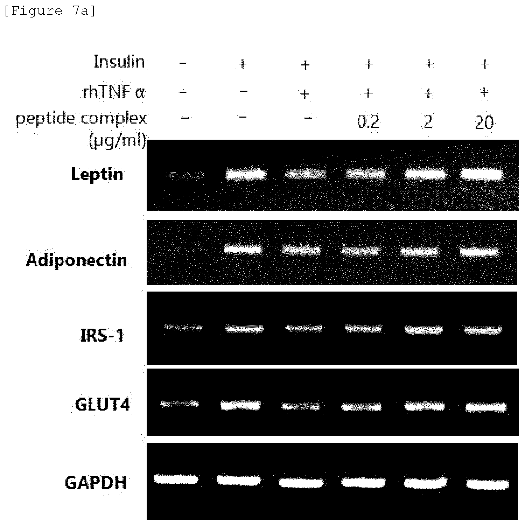

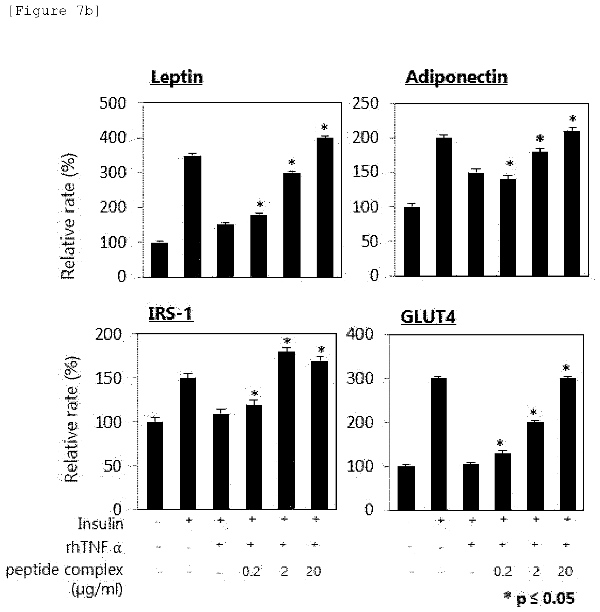

As shown in figures 7a and 7b, the experiment showed that the expressions of glucose uptake-related genes leptin, adiponectin, IRS-1, and GLUT4, which had been decreased by rhTNF-α treatment, in adipocytes, were increased by the peptide complex treatment.

### Experimental Example 8: Inhibition of ROS production induced by palmitate

Whether the peptide complex prepared in Preparation Example 1 inhibits reactive oxygen species (ROS) production induced by palmitate, a type of free fatty acid, was confirmed through intracellular ROS detection analysis (FACS) .

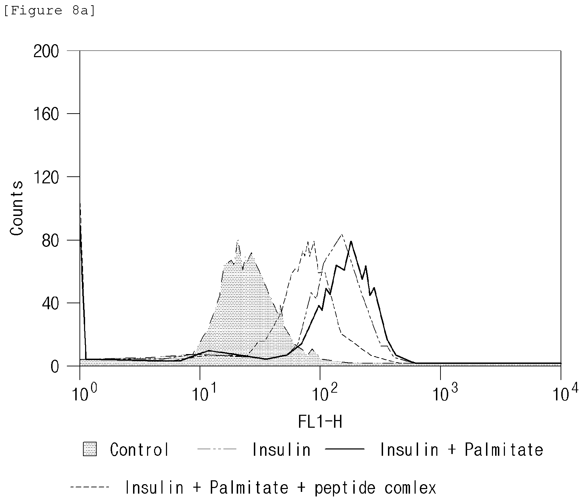

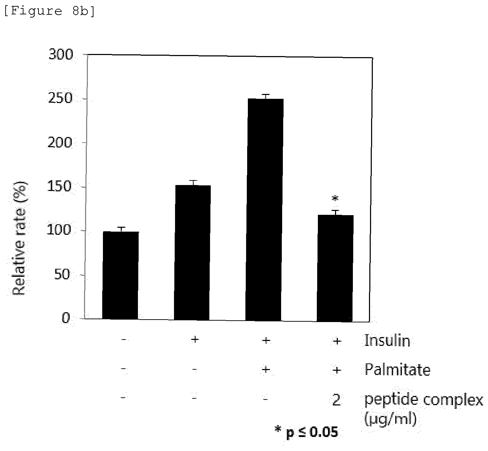

When 3T3-L1 preadipocytes reached 80% confluence, they were seeded in a 6-well plate at 5 x 10⁵ cells/well and used for the experiment. Two days after reaching confluence, the culture medium was replaced with a differentiation induction medium, and differentiation into adipocytes was induced. Two days after induction, preadipocytes were cultured for two days in a medium containing 10% FBS and 10 µg/mL insulin, and then the medium was replaced with a medium containing 10% FBS every two days until preadipocytes differentiated into adipocytes. When differentiation was completed, the medium was replaced with a serum-free medium, and cells were cultured for 4 hours to induce starvation. The cells were treated with rhTNF-α (2 nM), insulin (1 µg/ml), and the peptide complex (2 µg/ml) . An untreated control group and the treated group were cultured for 24 hours and treated with DCF-DH for 30 minutes. Then, FACS (Becton Dickinson & Company (BD), USA) was used to measure the oxidative activity from the fluorescence. As shown in the graphs of figures 8a and 8b, the measurement results showed that the amount of palmitate-induced reactive oxygen species (ROS) were reduced by the peptide complex.

### Experimental Example 9: Inhibition of TNF-α gene expression induced by palmitate

Whether the peptide complex prepared in Preparation Example 1 inhibits inflammation induced by palmitate, a type of free fatty acid, was confirmed through gene expression analysis (RT-PCR).

INS-1 cells, in which starvation was induced using serum-free media RPMI 1640 (Gibco, New York, USA), were pretreated with 25 µM palmitate for 2 hours and treated with the peptide complex (0.2, 2, and 20 µg/ml). Twenty-four hours later, RNA was extracted using the easy-BLUE^{™} Total RNA Extraction Kit (Qiagen, Germany). The extracted RNA was converted into cDNA using RT-PCR premix (iNtRON Biotechnology, Seongnam, Korea). A reaction mixture was prepared using PCR premix (iNtRON Biotechnology, Seongnam, Korea) and primers for TNF-α and GAPDH genes, and PCR was carried out using PCR machine (Eppendorf, Germany). Subsequently, mRNA expression patterns were determined by agarose gel electrophoresis. The nucleotide sequences of the primers used in the experiment are shown in Table 4 below.

**[Table 4]**

| Gene | Primer | Sequence (5' -> 3') | SEQ ID NO: |
|---|---|---|---|
| TNF-α | F | ATG AGC ACG GAA AGC ATG AT | 17 |
| | R | CTC TTG ATG GCA GAG AGG AG | 18 |
| GAPDH | F | GTG ATG GCA TGG ACT GTG GT | 9 |
| | R | GGA GCC AAA AGG GTC ATC AT | 10 |

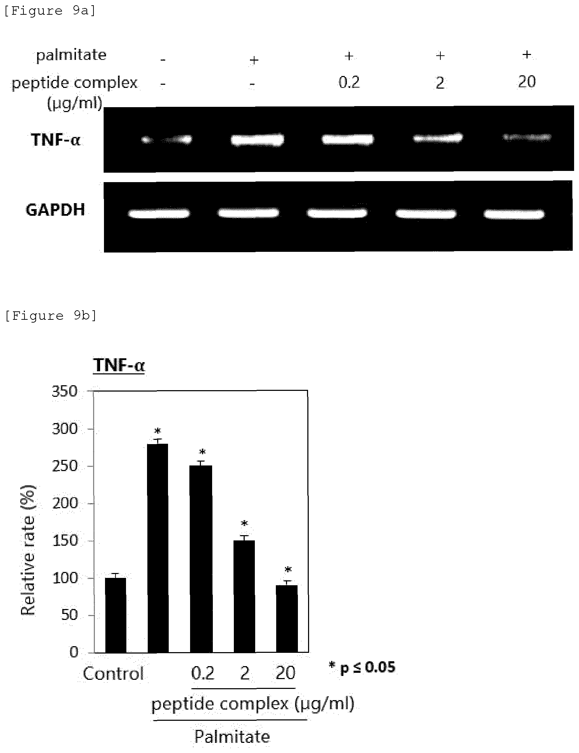

As shown in figures 9a and 9b, the experiment showed that the gene expression of TNF-α, an inflammatory protein induced by palmitate, was significantly reduced by the peptide complex treatment.

### Experimental Example 10: Inhibition of the expressions of inflammatory cytokines induced by palmitate

Whether the peptide complex prepared in Preparation Example 1 inhibits the expressions of inflammatory cytokines induced by palmitate, a type of free fatty acid, was confirmed through protein expression analysis (western blot analysis).

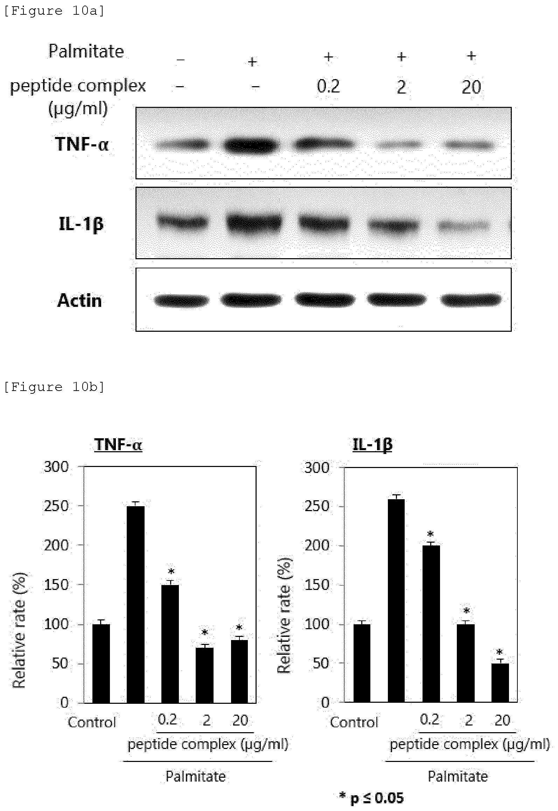

INS-1 cells (rat pancreatic beta cells), in which starvation was induced using serum-free media RPMI 1640 (Gibco, New York, USA), were pretreated with 25 µM palmitate for 2 hours and treated with the peptide complex (0.2, 2, and 20 µg/ml) . Twenty-four hours later, the cells were washed once with PBS, a lysis buffer (Millipore, Darmstadt, Germany) containing 10 mM Tris (pH 7.5), 100 mM NaCl, 1% NP-40, and a protease inhibitor was added to lyse the cells on ice for 30 minutes. Then, the lysate was centrifuged at 4°C and 13,000 rpm for 10 minutes. Thus obtained proteins were quantified using the BCA kit (Thermo Fisher Scientific, Waltham, USA). The same amount of protein was separated by sodium dodecyl sulfate-polyacrylamide gel electrophoresis (SDS-PAGE) and then and electrotransferred to a polyvinylidene difluoride membrane. In order to block nonspecific binding of the antibody, the membrane to which the protein had been attached was treated with 5% skim milk (BD, New Jersey, USA) and blocked for one hour, and then the primary antibody was reacted overnight at 4°C. The membrane was washed with PBS-T, the secondary antibody was reacted at room temperature for 1-2 hours, the membrane was washed again with PBS-T, and then the proteins were visualized on an X-ray film using enhanced chemiluminescence (ECL) (Thermo Fisher Scientific, Waltham, USA). The antibodies used in the experiment are as follows: anti-TNF-α antibody (Cell signaling technology (CST), USA), anti-IL-1β antibody (Cell signaling technology (CST), USA), and anti-actin antibody (Santa Cruz Biotechnology, USA). As shown in figures 10a and 10b, the experiment showed that the expression levels of the TNF-α and the IL-1β proteins increased by palmitate treatment in INS-1 cells (rat pancreatic beta cells) were again significantly reduced by the peptide complex treatment.

### Experimental Example 11: Inhibition of apoptosis of pancreatic beta cells induced by palmitate

Whether the peptide complex prepared in Preparation Example 1 inhibits apoptosis of pancreatic beta cells induced by palmitate, a type of free fatty acid, was confirmed through cell cytotoxicity assay.

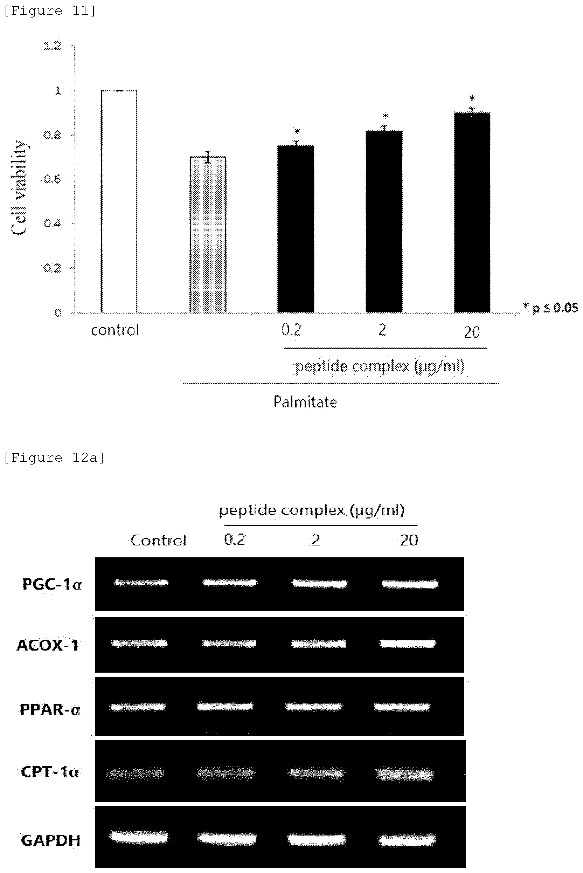

INS-1 cells (rat pancreatic beta cells), in which starvation was induced using serum-free media RPMI 1640 (Gibco, New York, USA), were pretreated with 25 µM palmitate for 2 hours and treated with the peptide complex (0.2, 2, and 20 µg/ml). Twenty-four hours later, CCK-8 solution (Dojindo, Kumamoto, Japan) was added to reach 1/10 of the volume of the culture medium, and the absorbance was measured every 30 minutes or 1 hour. When the absorbance value of the control group was 1.0 on average, the experiment was terminated. The absorbance of the reaction product was measured at the wavelength of 450 nm using a microplate reader. As shown in figure 11, the experiment showed that the increase in INS-1 cell death induced by palmitate treatment was again reduced by the peptide complex treatment, thereby increasing the cell viability.

### Experimental Example 12: Promotion of the expressions of glucose uptake-related genes

Whether the peptide complex prepared in Preparation Example 1 promotes the expressions of glucose uptake-related genes, peroxisome proliferator-activated receptor-gamma coactivator-1 alpha (PGC-1α), acyl-CoA oxidase 1 (ACOX-1), peroxisome proliferator-activated receptor-alpha (PPAR-α), or carnitine palmitoyltransferase 1 alpha (CPT-1α) was confirmed through gene expression analysis (RT-PCR).

When HepG2 hepatocellular carcinoma cells reached 80% confluence, they were seeded in a 12-well plate at 2 x 10⁵ cells/well and used for the experiment. The next day, the medium was replaced with a serum-free medium to induce starvation for 4 hours. Four hours later, the cells were treated with the peptide complex (0.2, 2, and 20 µg/ml). Twenty-four hours later, RNA was extracted from an untreated control group and the treated group, and RT-PCR was carried out. RNA was extracted using the easy-BLUE^{™} Total RNA Extraction Kit (Qiagen, Germany). The extracted RNA was converted into cDNA using RT-PCR premix (iNtRON Biotechnology, Seongnam, Korea). A reaction mixture was prepared using PCR premix (iNtRON Biotechnology, Seongnam, Korea) and a primer for PGC-1α, ACOX-1, PPAR-α, or CPT-1α genes, and PCR was carried out using PCR machine (Eppendorf, Germany). Subsequently, mRNA expression patterns were determined by agarose gel electrophoresis. The nucleotide sequences of the primers used in the experiment are shown in Table 5 below.

**[Table 5]**

| Gene | Primer | Sequence (5' -> 3') | SEQ ID NO: |
|---|---|---|---|
| PGC-1α | F | AGTCTGTATGGAGTGACATCGAG | 19 |
| | R | GGCAATCCGTCTTCATCCAC | 20 |
| ACOX-1 | F | CCGCCGAGAGATCGAGAAC | 21 |
| | R | CAGTTGCCTGGTGAAGCAAG | 22 |
| PPAR-α | F | AAGGGCTTCTTTCGGCGAAC | 23 |
| | R | TGACCTTGTTCATGTTGAAGTTCTTCA | 24 |
| CPT-1α | F | CCTCCAGTTGGCTTATCGTG | 25 |
| | R | TTCTTCGTCTGGCTGGACAT | 26 |
| GAPDH | F | GTG ATG GCA TGG ACT GTG GT | 9 |
| | R | GGA GCC AAA AGG GTC ATC AT | 10 |

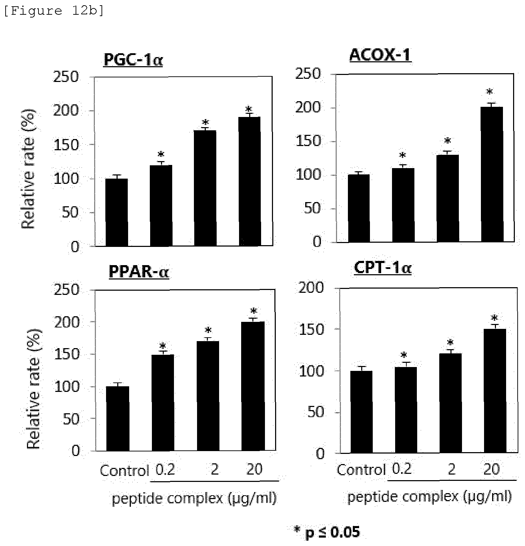

As shown in figures 12a and 12b, the experiment showed that the expression of PGC-1α, ACOX-1, PPAR-α, or CPT-1α genes, which are genes related to FFA beta-oxidation, was promoted by the peptide complex in HepG2 cells.

### Experimental Example 13: Promotion of lipolytic enzyme protein expressions in adipocytes

Whether the peptide of SEQ ID NO: 2 prepared in Preparation Example 1 promotes the expressions of lipolytic enzyme proteins in adipocytes was tested through protein expression analysis (western blot analysis).

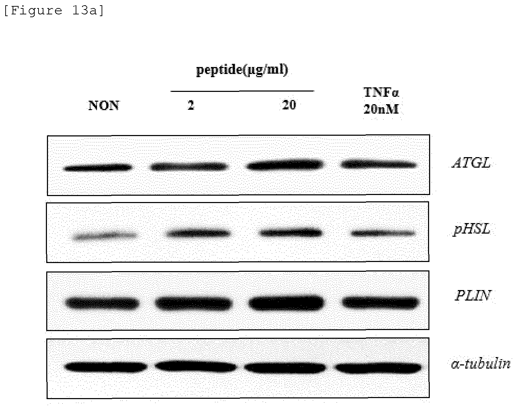

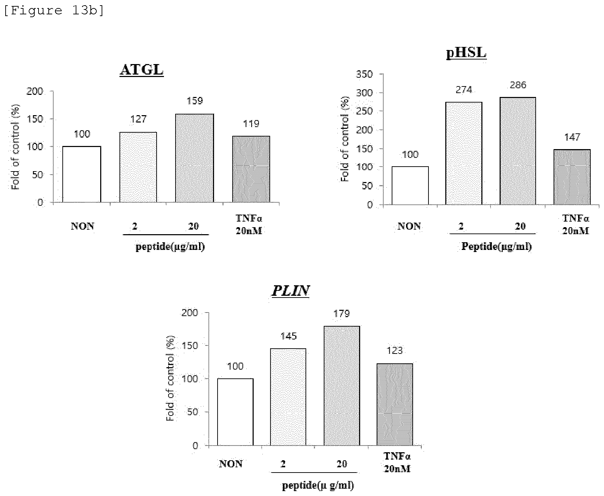

When 3T3-L1 preadipocytes reached 80% confluence, they were seeded in a 6-well plate at 5 x 10⁵ cells/well and used for the experiment. Two days after reaching confluence, the culture medium was replaced with a differentiation induction medium [DMEM medium containing 10% FBS, 1 µg/ml insulin, 0.5 mM isobutyl methyl xanthine (IBMX) (Sigma, USA), and 1 µM dexamethasone (Sigma, USA)], and differentiation into adipocytes was induced. Two days after induction, preadipocytes were cultured for two days in a medium containing 10% FBS and 10 µg/mL insulin, and then the medium was replaced with a medium containing 10% FBS every two days until preadipocytes differentiated into adipocytes. When differentiation was completed, the medium was replaced with a serum-free medium, and cells were cultured for four hours to induce starvation. Cells were divided into an untreated group (negative control), the peptide of Preparation Example 1 (2, 20 µg/ml) -treatment group, and the TNFα (20 nM)-treatment group (positive control), and then treated with the substances at predetermined concentrations and cultured for one hour. Then, the cells in each treatment group were lysed by adding a lysis buffer. After lysis, proteins obtained by centrifugation at 4°C and 12,000 rpm for 30 minutes were quantified using a BCA kit. Proteins were subjected to sodium dodecyl sulfate-polyacrylamide gel electrophoresis (SDS-PAGE) and electrotransferred to a membrane. The protein-attached membrane was blocked with 5% skim milk treatment, and then the primary antibody was reacted overnight at 4°C. The membrane was washed with PBS-T, the secondary antibody was reacted at room temperature for one hour, the membrane was washed again with PBS-T, and then visualization was carried out by using Gel Doc (Bio-Rad, Hercules, CA, USA) with a western detection reagent (Elpis Biotech, Daejeon, Korea). The antibodies used in the experiment are as follows: anti-ATGL antibody (Cell signaling technology (CST), USA), anti-pHSL antibody (Cell signaling technology (CST), USA), anti-PLIN antibody (Cell signaling technology (CST), USA), and anti-α-tubulin antibody (Santa Cruz Biotechnology, USA). As shown in figures 13a and 13b, the experiment showed that the treatment with the peptide of the present invention increased the expressions of lipolytic enzyme proteins, adipose triglyceride lipase (ATGL), phosphorylated hormone-sensitive lipase (pHSL), and perilipin (PLIN, lipid droplet-associated protein) in adipocytes.

### Experimental Example 14: Promotion of lipolysis in adipocytes

The release amount of glycerol, a lipolysis product, was analyzed to determine whether the peptide of SEQ ID NO: 2 prepared in Preparation Example 1 promotes lipolysis in adipocytes.

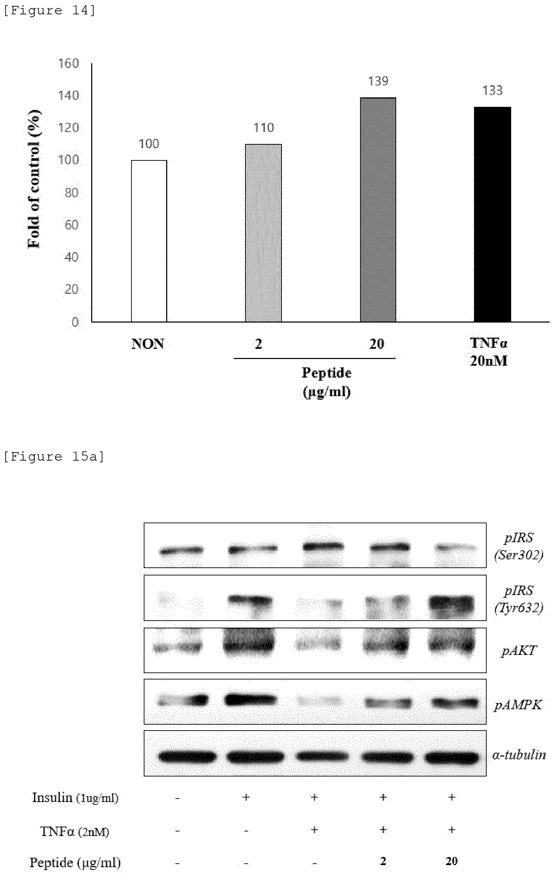

When 3T3-L1 preadipocytes reached 80% confluence, they were seeded in a 6-well plate at 5 × 10⁵ cells/well and used for the experiment. Two days after reaching confluence, the culture medium was replaced with a differentiation induction medium [DMEM medium containing 10% FBS, 1 ug/ml insulin, 0.5 mM isobutyl methyl xanthine (IBMX) (Sigma, USA), and 1 µM dexamethasone (Sigma, USA)], and differentiation into adipocytes was induced. Two days after induction, preadipocytes were cultured for two days in a medium containing 10% FBS and 10 µg/mL insulin, and then the medium was replaced with a medium containing 10% FBS every two days until preadipocytes differentiated into adipocytes. When differentiation was completed, the medium was replaced with a serum-free medium, and cells were cultured for four hours to induce starvation. Cells were divided into an untreated group (negative control), the peptide of SEQ ID NO: 2 of Preparation Example 1 (2, 20 µg/ml)-treatment group, and the TNF-α (20 nM)-treatment group (positive control), and then treated with the substances at predetermined concentrations and cultured for 48 hours. Then, the supernatants were obtained. Glycerol release assay was performed on the obtained supernatant using the "Glycerol Colorimetric assay kit" (Cayman Chemical, USA), and the amount of glycerol released was compared. As shown in figure 14, the glycerol release assay showed a tendency in which the release amount of glycerol, a lipolysis product, in adipocytes increased in a treated peptide concentration-dependent manner when adipocytes were treated with the peptide of the present invention.

### Experimental Example 15: Inhibition of an insulin-resistant signal and promotion of an insulin-sensitive signal

Protein expression was analyzed to determine whether the peptide of SEQ ID NO: 2 prepared in Preparation Example 1 inhibits insulin resistance and promotes insulin-sensitive signal transduction in adipocytes.

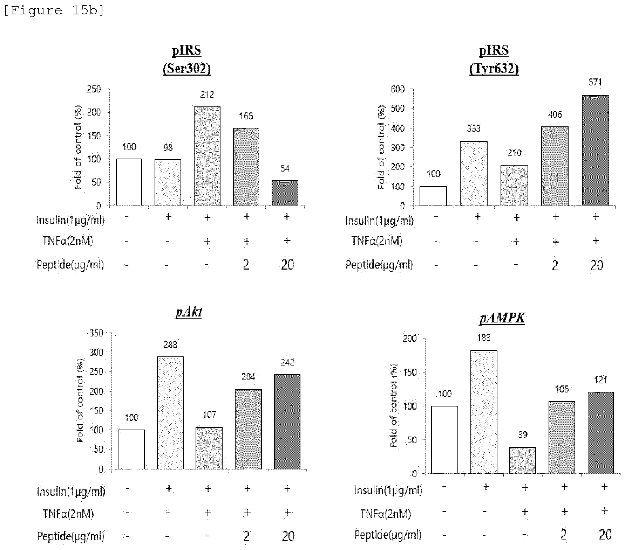

When 3T3-L1 preadipocytes reached 80% confluence, they were seeded in a 6-well plate at 5 × 10⁵ cells/well and used for the experiment. Two days after reaching confluence, the culture medium was replaced with a differentiation induction medium [DMEM medium containing 10% FBS, 1 µg/ml insulin, 0.5 mM isobutyl methyl xanthine (IBMX) (Sigma, USA), and 1 µM dexamethasone (Sigma, USA)], and differentiation into adipocytes was induced. Two days after induction, preadipocytes were cultured for two days in a medium containing 10% FBS and 10 µg/mL insulin, and then the medium was replaced with a medium containing 10% FBS every two days until preadipocytes differentiated into adipocytes. When differentiation was completed, the medium was replaced with a serum-free medium, and cells were cultured for four hours to induce starvation. To the cells, each of rhTNF-α (2 nM), insulin (1 µg/ml), and the peptide of SEQ ID NO: 2 of Preparation Example 1 (2, 20 µg/ml) was added and the cells were cultured for one hour. The cells were cultured for 30 minutes, the cells of each treatment group were lysed by adding a lysis buffer, and then centrifuged at 4°C and 12,000 rpm for 30 minutes. The obtained proteins were quantified using a BCA kit. Proteins were subjected to sodium dodecyl sulfate-polyacrylamide gel electrophoresis (SDS-PAGE) and electrotransferred to a membrane. The protein-attached membrane was blocked with 5% skim milk treatment, and then the primary antibody was reacted overnight at 4°C. The membrane was washed with PBS-T, the secondary antibody was reacted at room temperature for one hour, the membrane was washed again with PBS-T, and then visualization was carried out by using Gel Doc (Bio-Rad, Hercules, CA, USA) with a western detection reagent (Elpis Biotech, Daejeon, Korea). The antibodies used in the experiment are as follows: anti-phospho-IRS (Ser302) antibody (Cell signaling technology(CST), USA), anti-phospho-IRS (Tyr632) antibody (Cell signaling technology(CST), USA), anti-phospho-AKT antibody (Cell signaling technology(CST), USA), anti-phospho-AMPK antibody (Cell signaling technology(CST), USA), and anti-α-tubulin antibody (Santa Cruz Biotechnology , USA). As shown in figures 15a and 15b, the experiment showed that the treatment with the peptide of the present invention promoted the phosphorylation of Tyr632 of an insulin receptor substrate (IRS) and the activation of phosphorylation-AKT, which are factors for promoting insulin sensitivity. In addition, it was confirmed that when the peptide of the present invention was treated, the phosphorylation of AMPK, an insulin sensitivity-increasing signaling protein, increased. Meanwhile, it was confirmed that phosphorylation of Ser302 of an insulin receptor substrate (IRS) decreased under an insulin resistance-inducing environment by the treatment of the present peptide, and thus insulin resistance was inhibited.

### Preparation Example 2: Preparation of Pharmaceutical Compositions

### 2-1. Preparation of Powders

The peptide complex or the peptide of the present invention 2 g

### Lactose 1 g

The above ingredients were mixed and filled in an airtight pouch to prepare powders.

### 2-2. Preparation of Tablets

The peptide complex or the peptide of the present invention 100 mg
Corn starch 100 mg
Lactose 100 mg
Magnesium stearate 2 mg

The above noted ingredients were mixed and tabletted according to a conventional tablet preparation method to provide tablets.

### 2-3. Preparation of Capsules

The peptide complex or the peptide of the present invention 100 mg
Corn starch 100 mg
Lactose 100 mg
Magnesium stearate 2 mg

The above ingredients were mixed and filled in a gelatin capsule according to a conventional method for preparing capsules to prepare capsules.

### 2-4. Preparation of Pills

The peptide complex or the peptide of the present invention 1 g
Lactose 1.5 g
Glyserine 1 g
Xylitol 0.5 g

The above ingredients were mixed and prepared into a pill according to a conventional method in such a manner that one pill has a weight of 4 g.

### 2-5. Preparation of Granules

The peptide complex or the peptide of the present invention 150 mg
Soybean extract 50 mg
Glucose 200 mg
Starch 600 mg

The above ingredients were mixed, and 100 mg of 30% ethanol was added, followed by drying at 60°C. After the formation of granules, the granules were filled into packaging bags.

### Preparation Example 3: Preparation of Functional Food Compositions

### 3-1. Preparation of Health Food

The peptide complex or the peptide of the present invention 500 µg
Vitamin mixture suitable amount
Vitamin A acetate 70 mg
Vitamin E 1.0 mg
Vitamin D 0.13 mg
Vitamin B2 0.15 mg
Vitamin B6 0.5 mg
Vitamin B12 0.2 mg
Vitamin C 10 mg
Biotin 10 mg
Nicotinamide 1.7 mg
Folic acid 50 mg
Calcium pantothenate 0.5 mg
Mineral mixture suitable amount
Ferrous sulfate 1.75 mg
Zinc oxide 0.82 mg
Magnesium carbonate 25.3 mg
Monopotassium phosphate 15 mg
Dipotassium phosphate 55 mg
Potassium citrate 90 mg
Calcium carbonate 100 mg
Magnesium chloride 24.8 mg

Although ingredients relatively suitable for use in health foods are mixed in the composition ratio of the vitamin and mineral mixtures as a preferred example, the mixing ratio may be arbitrarily varied, and the ingredients may be mixed according to a general health food preparation method, and then prepared into granules, which may then be used for the preparation of a health food composition according to a general method.

### 3-2. Preparation of Health Drinks

The peptide complex or the peptide of the present invention 500 µg
Citric acid 1000 mg
Oligosaccharides 100 g
Green plum concentrate 2 g
Taurine 1 g
Add purified water to total volume of 900 ml

The above ingredients were mixed according to a general health drink preparation method, the mixture was heated and stirred at 85°C for about 1 hour to prepare a solution, the solution was filtered, the filtrate was collected in a sterilized container and then sealed and sterilized, followed by refrigerated storage, which was then used for preparing a heath drink composition. Although ingredients relatively suitable for use in favorite beverages are mixed in the above composition ratio as a preferred example, the mixing ratio may be arbitrarily varied depending on local and national preferences, such as demand classes, demand countries, purposes of use, and the like.

Although representative embodiments of the present application have been described above, the scope of the present application is not limited to the specific embodiments as described above, a person skilled in the art can change the present application within the scope described in the claims of the present application.

## Claims

1. A peptide complex comprising: (i) a peptide comprising the amino acid sequence of SEQ ID NO: 1; and (ii) a peptide comprising the amino acid sequence of SEQ ID NO: 2.

2. The peptide complex of claim 1, which has antidiabetic activity.

3. A pharmaceutical composition for preventing or treating diabetes, comprising the peptide complex of claim 1 as an active ingredient.

4. The pharmaceutical composition for preventing or treating diabetes of claim 3, wherein the peptide complex promotes glucose uptake.

5. The pharmaceutical composition for preventing or treating diabetes of claim 3, wherein the peptide complex inhibits an insulin-resistant signal or promotes an insulin-sensitive signal.

6. The pharmaceutical composition for preventing or treating diabetes of claim 3, wherein the peptide complex inhibits phosphorylation of Ser302 of an insulin receptor substrate (IRS) or phosphorylation of c-Jun N-terminal kinase (JNK).

7. The pharmaceutical composition for preventing or treating diabetes of claim 3, wherein the peptide complex inhibits the expressions of the TNF-α gene, the mammalian target of rapamycin (mTOR) gene, or the p70S6K gene under an insulin resistance-inducing environment.

8. The pharmaceutical composition for preventing or treating diabetes of claim 3, wherein the peptide complex enhances phosphorylation of Tyr632 of an insulin receptor substrate (IRS) or promotes activation of phosphoinositide 3-kinase (PI3K), activation of AKT, or activation of AMPactivated protein kinase (AMPK).

9. The pharmaceutical composition for preventing or treating diabetes of claim 3, wherein the peptide complex promotes the expressions of one or more genes selected from the group consisting of Leptin, Adiponectin, insulin receptor substrate 1 (IRS-1), glucose transporter type 4 (GLUT4), PGC-1α, ACOX-1, PPAR-α, and CPT-1α.

10. The pharmaceutical composition for preventing or treating diabetes of claim 3, wherein the peptide complex inhibits the production of reactive oxygen species (ROS), the expression of the TNF-α gene, the expression of the TNF-α protein, or the expression of the IL-1β protein, which are induced by free fatty acids, or inhibits apoptosis of pancreatic beta cells induced by free fatty acids.

11. A functional food composition for controlling blood glucose levels, comprising the peptide complex of claim 1 as an active ingredient.

12. The functional food composition for controlling blood glucose levels of claim 11, wherein the peptide complex promotes glucose uptake.

13. The functional food composition for controlling blood glucose levels of claim 11, wherein the peptide complex inhibits an insulin-resistant signal or promotes an insulin-sensitive signal.

14. A peptide comprising the amino acid sequence of SEQ ID NO: 2.

15. The peptide of claim 14, which has anti-diabetic or anti-obesity activity.

16. A pharmaceutical composition for preventing or treating diabetes, comprising the peptide of claim 14 as an active ingredient.

17. The pharmaceutical composition for preventing or treating diabetes of claim 16, wherein the peptide inhibits an insulin-resistant signal or promotes an insulin-sensitive signal.

18. The pharmaceutical composition for preventing or treating diabetes of claim 16, wherein the peptide has the activity of promoting phosphorylation of Tyr632 of an insulin receptor substrate (IRS), the activity of promoting activation of phospho-AKT, or the activity of promoting phosphorylation of AMPK; or
the peptide has the activity of inhibiting phosphorylation of Ser302 of an IRS under an insulin resistance-inducing environment.

19. A pharmaceutical composition for preventing or treating obesity, comprising the peptide of claim 14 as an active ingredient.

20. The pharmaceutical composition for preventing or treating obesity of claim 19, wherein the peptide promotes lipolysis in adipocytes.

21. The pharmaceutical composition for preventing or treating obesity of claim 19, wherein the peptide increases the expressions of lipolytic enzyme proteins, adipose triglyceride lipase (ATGL), phosphorylated hormone-sensitive lipase (pHSL), or perilipin (PLIN, lipid droplet-associated protein) in adipocytes.

22. A functional food composition for controlling blood glucose levels, comprising the peptide of claim 14 as an active ingredient.

23. The functional food composition for controlling blood glucose levels of claim 22, wherein the peptide inhibits an insulin-resistant signal or promotes an insulin-sensitive signal.

24. A functional food composition for preventing or alleviating obesity, comprising the peptide of claim 14 as an active ingredient.

25. The functional food composition for preventing or alleviating obesity of claim 24, wherein the peptide promotes lipolysis in adipocytes.
